# EUROPEAN PATENT APPLICATION

(11) **EP 2 977 017 A1**
(43) Date of publication of application: **27.01.2016**
(21) Application number: 14460042.6
(22) Date of filing: 22.07.2014
(51) Int. Cl.: A61B 17/70, A61B 17/86

(54) **Pedicle anchor assembly**

(71) Applicant: LfC sp. z o.o., 65-364 Zielona Gora (PL)
(72) Inventor: Ciupik, Lechoslaw Franciszek, 65-364 Zielona Gora (PL); Cecek, Jacek Michal, 65-001 Zielona Gora (PL)

(57) **Abstract**

A pedicle anchor assembly is provided. The pedicle screw assembly comprises:
- a bone anchor (1) including a bone engaging portion (3) with a distal end and a proximal end, and a head (5) located at the proximal end of the bone engaging portion (3), where the head (5) includes two diametrically opposite sides with flat head faces (7) and has a first head width (d1) in a first direction extending between the flat head faces (7) and a second head width (d2) in a second direction perpendicular to the first direction, the second head width being larger than the first head width, and
- a housing (13) extending along an axial direction and having an open axial end (14), where the housing (13) includes a bottom section (15) with a seat for the head (5) of the bone anchor (1) and with an axial bore (23) which allows the engaging portion (3) to extend from the head (5) through and out of the axial bore (23), the axial bore (23) having a bore width smaller than at least the second head portion width (d2), and where the housing (13) includes a jacket wall section (17) which extends in axial direction from ore close from the bottom section (15) to the open axial end (14) and which includes slits (19) extending axially in the jacket wall section (17) from the bottom section (15) to the open axial end (14), where the slits (19) are located on diametrically opposite sides of the jacket wall section (17),
- a washer (125) with dimensions allowing it to be inserted into the housing (13) through the axial open end (14), the washer (125) including two axial extensions (131) which extend from a first side of the washer (125) in axial direction of the housing (13) when the washer (125) has been placed in the housing, the two axial extensions (131) each having an outer face (35) showing away from the respective other axial extension (131) and a flat inner face (33) showing towards the respective other axial extension (131), where the distance between the flat inner faces (33) of the axial extensions (131) is adapted to the first head width (d1) so that the flat head faces (7) can be held and guided by the flat inner faces (33) of the axial extensions (131) and where the shapes of and the distance between the outer faces (35) of the axial extensions (131) correspond to the shape and width of the axial bore (23) in the bottom section (15) of the housing (13); and
- cooperating first and second snap-in connection elements (137, 141) where the first snap-in connection elements (137) are located at the washer (125) and at least one second snap-in connection element (143) is located at the housing (13).

## Description

The present invention relates to pedicle anchor assemblies.

A typical bone anchor assembly comprises a bone anchor and a housing which extends along an axial direction and has an axial open end. The bone anchor includes a bone engaging portion with a proximal end and a distal end, and a head located at the proximal end of the engaging portion. The housing includes a bottom section and a jacket wall section which extends in axial direction from the bottom section to the axial open end of the housing. The bottom section is equipped with a bore heaving a bore width smaller than the width of the head of the bone anchor. The bore allows the engaging portion of the bone anchor to extend from the head through and out of the bore. Moreover, the jacket wall of the housing comprises slits extending in axial direction from to the axial open end of the housing. These slits are located on diametrically opposite sides of the jacket wall so as to allow for inserting a rod into the housing through the open axial end such that the rod extends through and out of the cylindrical housing in a direction perpendicular to the axial direction. In addition, the open axial end can typically be closed by means of plug with a thread cooperating with a thread of the housing. Examples for such pedicle anchor assemblies are disclosed in WO 91/16020 A1, WO 97/40762 A1, WO 98/27884 A1, WO 03/024343 A1, WO 2011/133690 A2, US 5, 545,165 , US 2013/0226243 A1, US 2013/0253589 A1, US 2013/0268006 A1, and PL 214656 B1.

Sometimes, the shape of the underside of the plug is adapted to the shape of the rod, as it is the case in, US 2013/0226243 A1, US 2013/0253589 A1, US 2013/0268006 A1, and PL 214656 B1. In addition, washers may be present between the rod and the head of the bone anchor which have surfaces that are adapted to the shape of the rod to be held. Such washers are, for example, disclosed in WO 98/27884 A1, US 2011/133690 A2, US 2013/0226243 A1, US 2013/0253589 A1, and US 2013/0268006 A1. To fix the washers inside the housing the washers may be provided with snap-in connections, as it is the case with the washers disclosed in US 2013/0268006 A1.

Sometimes the heads of the bone anchors are ball shaped so as to allow for adjusting the direction in which the anchor extends with respect to the axial direction of the housing, given that the axial bore in the bottom section of the housing has appropriate dimensions. Examples of ball shaped heads are disclosed in WO 91/16020 A1, WO 98/27884 A1, and US 2013/0268006 A1. In other pedicle anchor assemblies, such as those described in WO 2011/133690 A2 and US 2013/0226243 A1, the allowed movement of the bone anchor is restricted to a movement within a single plane. In WO 2011/133690 A2 the movement of the bone anchor is restricted by pins that extend in openings on opposite sides of the bone anchor head. In US 2013/0226243 A1 the movement of the bone anchor is restricted by giving the head of the bone anchor two diametrically opposite flat faces which are guided by guiding faces of an element that is inserted into the underside of the housing and has an upper spherical surface mating with a lower spherical surface of a washer fixed in the housing by means of pins. Both solutions are complex, both in production and in handling, since they compromise a considerable number of parts.

With respect to the mentioned state of the art it is an objective of the present invention to provide an advantageous pedicle anchor assembly in which the movements of the bone anchor are restricted to a single plane.

This objective is achieved by a pedicle anchor assembly as claimed in claim 1. The depending claims contain further developments of the invention. An inventive pedicle anchor assembly comprises a bone anchor which is designed to anchor the assembly in a bone structure. The bone anchor includes a bone engaging portion, e.g. a screw, a hook or a harpoon like structure, with a distal end and a proximal end, and a head located at the proximal end of the engaging portion. The head, which may be equipped with a socket for a driving key if the bone anchor is implemented as a screw, includes two diametrically opposite sides with flat head faces and has a first head width in a first direction extending between the flat head faces and a second head width in a second direction perpendicular to the first direction. The second head width is larger than the first head width.

In addition, the inventive pedicle anchor assembly comprises a housing that extends along an axial direction and has an open axial end. The housing may, in particular, be a cylindrical housing. In the context of the present invention, being cylindrical shall not only mean a cylinder with a circular cross section but also a cylinder with a different cross section, such as, e.g., a cylinder with an elliptic cross section, a cylinder with a polygonal cross section, or even a cylinder with an irregular cross section. The housing is adapted to house the head of the bone anchor and to receive a rod through the open axial end. It includes a bottom section with a seat for the head portion of the bone anchor and with an axial bore which allows the engaging portion of the bone anchor to extend from the head portion through and out of the bore. The axial bore has a bore width that is smaller than at least the second head width. In addition, the housing includes a jacket wall section which extends in axial direction from the bottom section to the open axial end and which includes slits extending axially in the wall section from or from close to the bottom section to the open axial end. These slits are located on diametrically opposite sides of the jacket wall so as to allow a rod to be inserted into the housing through the open axial end and to extend through and out of the housing in a direction perpendicular to the axial direction.

Furthermore, the inventive pedicle anchor assembly comprises a washer with dimensions allowing it to be inserted into the housing through the axial open end. This washer, which preferably has a first side with a shape that is adapted to fit to the head portion of the bone anchor, includes two axial extensions which extend from the first side of the washer in axial direction of the housing when the washer has been placed in the housing. These axial extensions each have an outer face showing away from the respective other axial extension and a flat inner face showing towards the respective other axial extension. The distance between the flat inner faces of the axial extensions is adapted to the first head width so that the flat head faces can be held and guided by the flat inner faces of the axial extensions. The shapes of and the distance between the outer faces of the axial extensions correspond to the shape and width of the bore in the bottom section of the housing such that two axial extensions can extend into or through the axial bore. If, for example, the axial bore has a circular cross section the outer faces of the axial extensions would be curved in a direction perpendicular to the axial direction of the housing. Moreover, if the head of the bone anchor equipped with a socket for a driving key the washer has a hole that allows a driving key to reach the socket through the washer.

The inventive pedicle anchor assembly further comprises cooperating first and second snap-in connection elements where the first snap-in connection elements are located at the washer and at least one second snap-in connection element is located at the housing.

In particular, the axial extensions of the washer may be resilient axial members and the first snap-in connection elements may be located at the outer faces of these resilient axial members. The at least one second snap-in connection element would then be located at the axial bore. For example, the first snap-in connection elements may be located in an axial middle section of the outer faces of the resilient axial members or at an axial end of the outer faces of the resilient axial members. In the first case, the at least one second snap-in connection element would be located at an inside wall of the axial bore, whereas the at least one second snap-in connection element would be located at or formed by the rim of the axial bore in the second case. Furthermore, the first snap-in connection elements may be projections extending from the outer faces of the resilient axial members or recesses in the outer faces of the resilient axial members. In the first case, the least one second snap-in connection element would be a recess in the inside wall of the axial bore, whereas the least one second snap-in connection element would be a projection extending from the inside wall of the axial bore in the second case. Such a projection could be located in an axial middle section of the bore wall or at the rim of the bore wall.

The present invention provides a pedicle bone anchor assembly which allows for restricting the movement of the bone anchor such that the bone anchor can only move within a defined plane and which achieves this restriction with only three elements, namely a bone anchor with a screw head with flat side faces, a housing with an axial bore and a washer with axial extensions. By providing the washer with snap-in connection elements that cooperate with snap-in connection elements of the housing the washer can easily be fixed without any special tool just by introducing the washer into the housing through the axial open end and pressing it towards the bottom section of the housing until the snap-in connection snaps in. Moreover, the low number of parts simplifies production and, thus, reduces costs of the pedicle anchor assembly.

In an advantageous development of the inventive pedicle anchor assembly the washer has a second side with a shape that is adapted to the shape of the rod to be received by the housing. By this measure, a situation in which the rod might be weakened by high loads acting on small local areas of the rod can be reduced.

In a further development of the inventive pedicle anchor assembly cylindrical or spherical faces extend between the flat faces of the head of the bone anchor, where the cylindrical or spherical faces are equipped with an external thread. In case of cylindrical faces, these faces are coaxial with the axial direction of the housing. The housing is equipped with an internal thread at the inside of the jacket wall. This internal thread begins at the open axial end of the housing and extends to a location at a distance from the bottom section of the housing with a thread free section being formed between the internal thread and the bottom section. In this design the bone anchor can be screwed through the housing with its engaging portion ahead until the head reaches the thread free section. In particular, the aperture and the axial extension of the thread free section within the jacket wall of the housing may be larger than the second head width and an axial extension of the head, respectively. In this case, once the thread free section is reached the bone anchor can be pivoted within a certain range and is, at the same time, secured against slipping out of the housing. The pivoting range is larger for spherical faces than for cylindrical faces. However, with spherical faces only a smaller part of the external thread engages the internal thread of the housing.

During Implantation of a pedicle anchor assembly it is advantageous if the housing has a certain minimum length in axial direction. On the other hand, when implanted, the housing should be as short as possible to reduce a risk of irritating surrounding tissue. Therefore, according to an advantageous development, the jacket wall of the housing is equipped with a predetermined breaking line along its circumference which breaking line is located in an axially mid-section of the jacket wall. The predetermined breaking line allows for braking off parts of the housing after implantation in order to shorten it.

The pedicle anchor assembly may further comprise a plug element which includes a pin with an external thread that corresponds to the internal thread of the jacket wall section of the housing. The pin allows for moving the plug element axially within the housing by rotating the pin. In particular, the plug element may include two opposing wall sections and a traverse connecting the wall sections, where the wall sections are dimensioned such that they fit into the slits of the jacket wall section. In this case, the pin is rotatably secured to the traverse. By means of such a plug, a force can be exerted onto a rod extending through the housing between the washer and the plug so as to secure the rod against slipping of the housing. To prevent from high loads acting on small localized areas of the rod, and thus from weakening the rod, the traverse may have an underside with a shape that is adapted to the shape of the rod.

Further features, properties and advantages of the present invention will become clear from the following description of embodiments in conjunction with the accompanying drawings.
Figure 1 shows a bone anchor used in embodiments of an inventive pedicle anchor assembly.
Figure 2 shows a housing used in embodiments of an inventive pedicle anchor assembly.
Figure 3 shows a washer of a first embodiment of the inventive pedicle anchor assembly in a first perspective view.
Figure 4 shows the washer of figure 3 in a second perspective view.
Figure 5 shows an inventive pedicle anchor assembly in a perspective view.
Figure 6 shows a washer of a second embodiment of the inventive pedicle anchor assembly in a perspective view.
Figure 7 shows the pedicle anchor assembly according to the second embodiment in a sectional view.
Figure 8 shows a detail of figure 7.
Figure 9 shows a washer of a third embodiment of the inventive pedicle anchor assembly.
Figure 10 shows the pedicle anchor assembly according to the third embodiment in a sectional view.
Figure 11 shows a detail of figure 10.
Figure 12 shows an inventive pedicle anchor assembly with a plug in an exploded view.
Figure 13 shows the pedicle anchor assembly in an assembled state.
Fig. 14 shows a further embodiment of the inventive pedicle anchor assembly in an assembled state.
Fig. 15 shows a still further embodiment of the inventive pedicle anchor assembly in an assembled state.

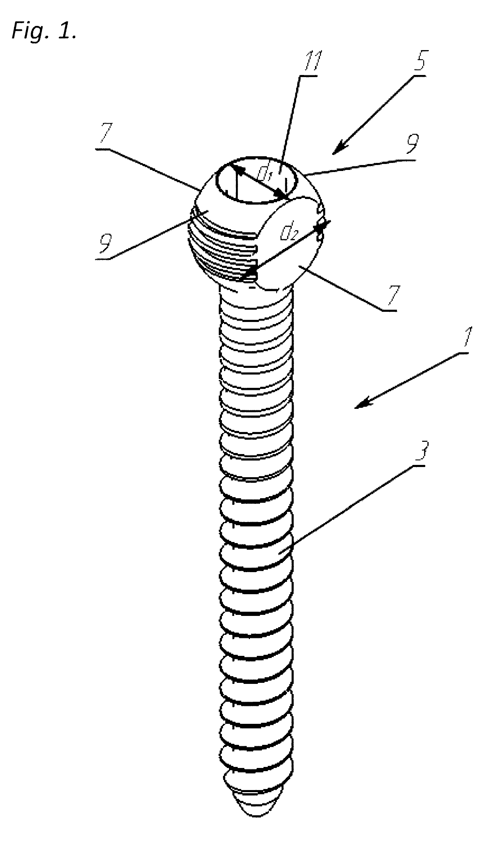

A first embodiment of the inventive pedicle anchor assembly will be explained with reference to figures 1 to 5, of which figure 1 shows the bone anchor of the pedicle anchor assembly. In the present embodiment, the bone anchor 1 includes a bone engaging portion 3 which is implemented as a screw and a head 5 which includes two flat faces 7 which are connected by a spherical zone 9, except for the location where the bone engaging portion 3 meets the head 5. In other words, the head is implemented as a ball with two diametrically opposite flat faces 7. An external thread which is intermitted by the flat feces 7 is cut into the spherical segment 9. Moreover, the head 5 may be equipped with a socket 11 for a screw driver or any other suitable driving tool for driving the bone anchor, as it is shown in Figure 1.

Due to the geometry of the head 5 the head has a first head width d1, which is the width between the flat faces 7. This first head width d1 is smaller than the diameter of the spherical zone 9. The diameter of the spherical zone 9 can be regarded as a second head width d2.

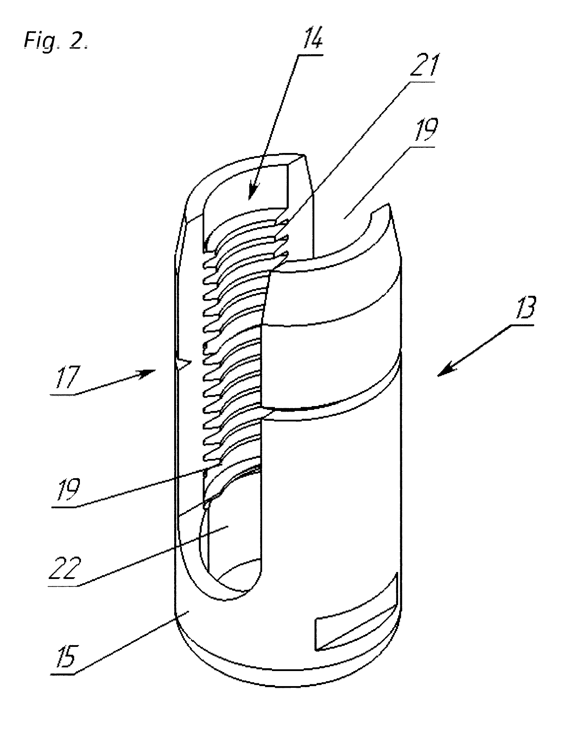

Figure 2 shows a cylindrical housing 13 of the first embodiment of the inventive pedicle anchor assembly. In the present embodiment, the cylindrical housing 13 has a spherical cross section. However, in other embodiments the outside cross section of the cylindrical housing may differ from a spherical cross section. For example, the outside cross section of the housing may be quadratic, hexagonal or of any other polygonal shape. Alternatively, it may have an elliptical cross section or even an irregular cross section.

The cylindrical housing 13 has an open axial end 14 and a bottom section 15. A jacket wall section 17 extends from the bottom section 15 to the open axial end 14. Slits 19 are formed in the jacket wall section 17 which extend along the axial direction of the housing from the bottom section to the open axial end 14. These slits are located in diametrically opposite sections of the jacket wall 17 and allow for receiving and accommodating a rod that extends through the cylindrical housing 13 in a radial direction of the housing. Moreover, the inside face of the jacket wall section 17 is equipped with an internal thread 21 that corresponds to the external thread of the spherical zone 9 of the bone anchor head 5 so that the bone anchor head 5 can be moved through the housing 13 in axial direction by rotating the bone anchor 1 about its longitudinal axis.

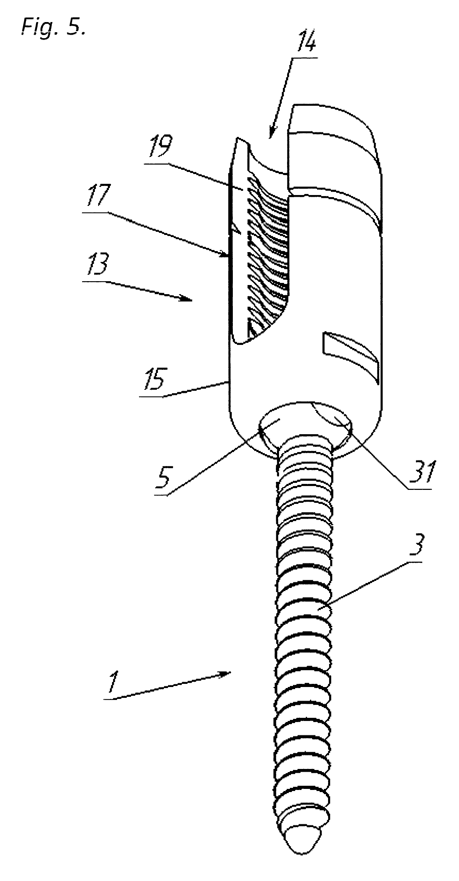

The bottom section 15 of the housing 13 includes an axial bore 23 which is visible in figure 5. The screw 3 of the bone anchor 1 can extend though this axial bore 13 so as to project over the bottom section 15 of the housing 13. Moreover, the internal thread 21 does not extend to the bottom section 15 but ends at a distance from it. The distance is chosen such that the bone anchor head 5 can rotate in the bottom section 15 so that the screw portion 3 of the bone anchor can be pivoted in a certain angular range since the diameter of the axial bore 23 is larger than the diameter of screw portion 3 of the bone anchor 1.

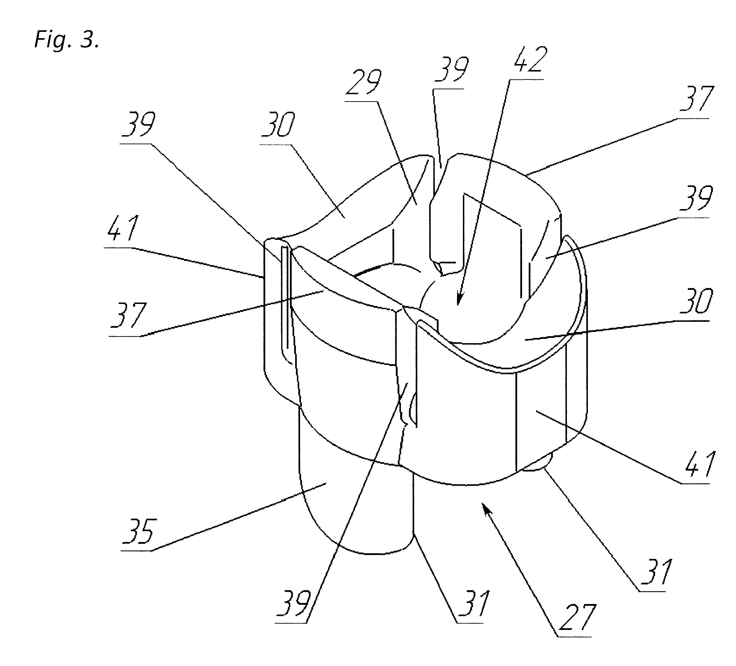

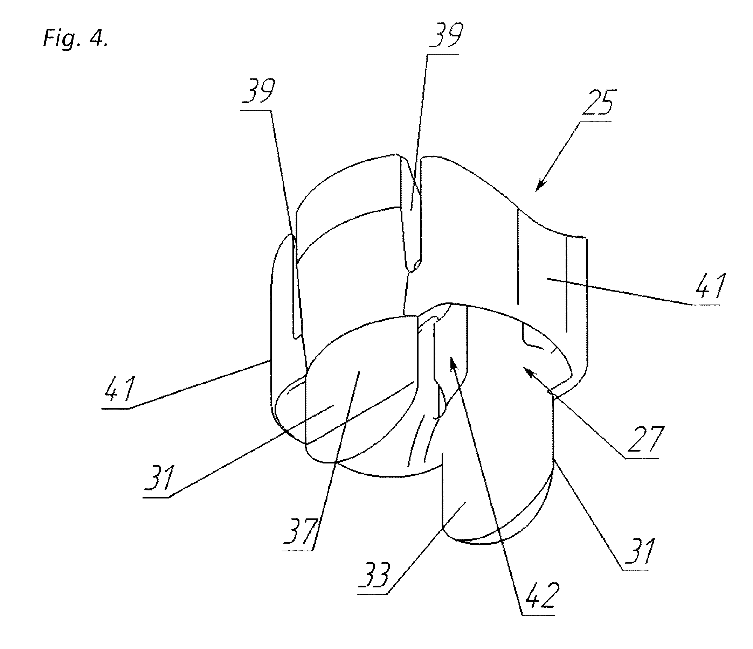

The pedicle anchor assembly further comprises a washer 25 which is shown in figures 3 and 4 in two different perspective views. The washer 25 comprises an lower side 27 that is, in the present embodiment, adapted to the shape of the head 5 of the bone anchor 1 and an upper side 29 which has a curved shape the curvature of which is adapted to the curvature of the rod to be received by the housing 13. Moreover, the washer 25 comprises two axial extensions extending from the lower side 27 in axial direction of the housing 13 when the washer 25 is inserted into the housing. The axial extensions 31 each have a flat inner face 33 showing towards the opposing axial extension 31 and an outer face 35 showing away from the opposing axial extension 31. The inner faces 33 are flat faces and are spaced from each other by a distance that allows to receive the head 5 of the bone anchor 1 in an orientation with its flat faces 7 showing towards the flat inner faces 33 of the extensions 31. Hence, the flat inner faces 31 serve as guide faces which prevent the bone anchor 1 from rotating along its longitudinal axis and guide the bone anchor such that it can only pivot in a plane which is parallel to the flat inner faces 33 of the axial extension 31.

In the present embodiment, the outer faces 35 of the axial extensions are curved with a curvature that corresponds to the curvature of the inner wall of the axial bore 23. Hence, the axial extensions 31 can extend into the axial bore 23 as it can be seen in figure 5.

The washer 25, which is made of a generally resilient material, also comprises snap-in connection elements 37 which are formed by resilient wall sections in the present embodiment. These resilient wall sections are formed by dividing them from other wall sections by means of slits 39. Due to their resilience these snap-in connection elements 37 can bend towards each other to reduce the outer diameter of the washer so that the washer can be introduced into the housing 13 through the open axial end 14. Once the washer reaches the thread free inner section 22 of the housing 13, which can be seen as a recess as compared to the threaded section of the housing, the wall sections 37 expand again and snap-in to the recess formed by the thread free section of the housing. Hence, the washer 25 can easily be secured inside the housing 13 by just pressing it through the housing in axial direction until it reaches the thread free inner section 22.

In the present embodiment, the washer 25 also comprises side walls 41 which extend into the slits 19 of the housing 13 when the washer 25 is inserted into the housing. Hence, the washer 25 cannot rotate about the axial direction of the housing 13 when it is introduced into the housing. The upper side 30 of these side walls has a curvature which is adapted to the curvature of the rod to be received by the housing 13. In addition, the washer 25 has an inner open section 42 which allows the tool for driving the bone anchor to reach through the washer 25 to the socket 11 of the bone anchor head 5.

The pedicle anchor assembly in its assembled state is shown in figure 5, which shows a perspective view of the assembly.

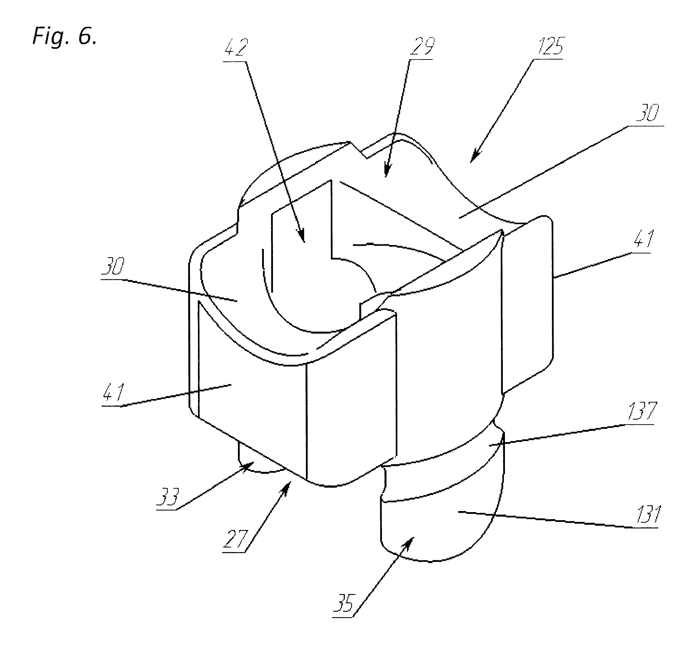

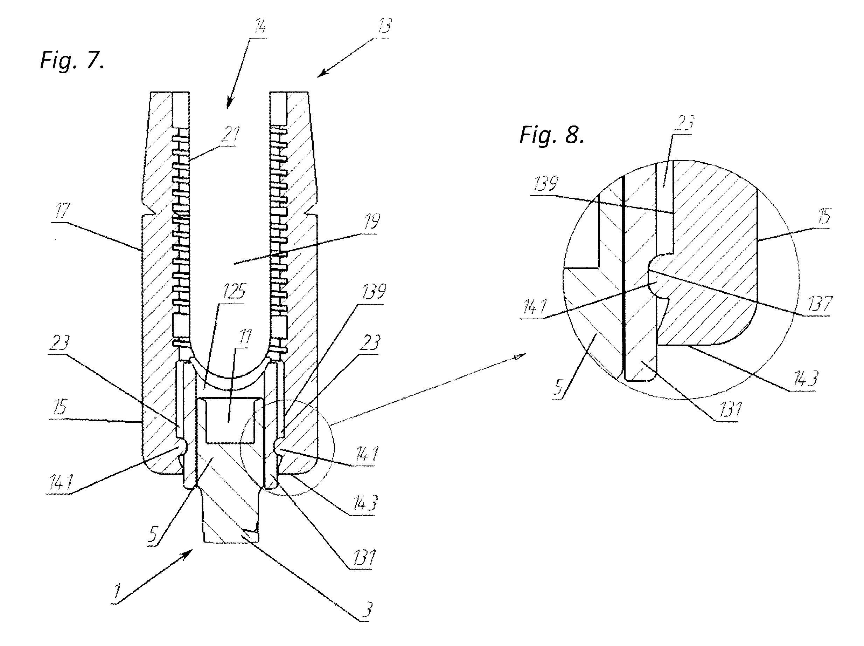

A second embodiment of the inventive pedicle anchor assembly will be described with respect to figures 6 to 8. In these figures, figure 6 shows the washer 125 of the second embodiment and figure 7 shows the pedicle anchor assembly in a sectional view. Figure 8 shows a detail of figure 7. The basic design of the second embodiment is very similar to the design of the first embodiment. The differences between both embodiments lie in the design of the axial extensions 131 of the washer 125 and the cooperating first and second snap-in connection elements 137, 141. Elements of the second embodiment which do not differ from the first embodiment are denominated by the same reference numerals as in figures 1 to 5 and will not be described again to avoid unnecessary repetitions.

As already mentioned, the main differences between the first embodiment and the second embodiment lie in the design of the washer 125 and the snap-in connection elements 137, 141. A washer 125 as used in the second embodiment of the pedicle anchor assembly is shown in figure 6. The washer 125 comprises an upper side 29 with a surface 30 having a curvature that is adapted to the curvature of a rod to be received by the housing 13 of the pedicle anchor assembly. Moreover, the washer 125 comprises a lower side 27 that is adapted to fit onto the bone anchor head 5 and two axial extensions 131 which extend from the lower side 27 in an axial extension of the housing 13 when the washer 125 is inserted into the housing 13. These axial extensions 131 form resilient axial members, which are resilient due to their shape and due to the resilience of the material the washer 125 is made of. Note in this context that a typical material of the washer, and also of the housing and the bone anchor, is titanium. However, also other resilient biocompatible materials or combinations of such materials could be used.

Like in the first embodiment, the axial extensions 131 have flat inner faces 33 which show towards each other and form a space between them. Moreover, also like in the first embodiment, the axial extensions 131 have outer faces 35 which are curved with a curvature that is adapted to the curvature of the wall 139 of the axial bore 23 in the bottom section 15 of the housing 13. In the present embodiment, recesses 137 are present in the outer faces 35 of the axial extensions 131. These recesses extend in circumferential direction of the curved outer surfaces 35 and are located in an axial middle section of the axial extensions 131. At the same time, the inner wall 139 of the axial bore 23 is equipped with a projection 141 that extends along the circumferential direction of the wall at a distance from the rim 143 of the bore 23.

When the washer 125 is inserted into the housing 13 through the axial open end 14 sidewalls 41 of the washer 125 extend into the slits 19 of the housing so that the orientation of the washer is fixed. Once the washer 125 reaches the bottom section 15 of the housing 13 the axial extensions 131 will be bent towards each other by the circumferential projection 141 at the bore wall 139. When the washer 125 is further moved towards the bottom section 15 of the housing 13 the recesses 137 in the outer faces 35 of the axial extensions 131 will eventually reach the circumferential projection 141 at the bore wall 139 so that the projectionssnap back. Thereby, the circumferential projection 141 comes to rest in the recesses 137.

Like in the first embodiment, the inner faces 33 of the axial extensions 131 serve for holding and guiding the head 5 of the bone anchor 1, as it is shown in figure 7.

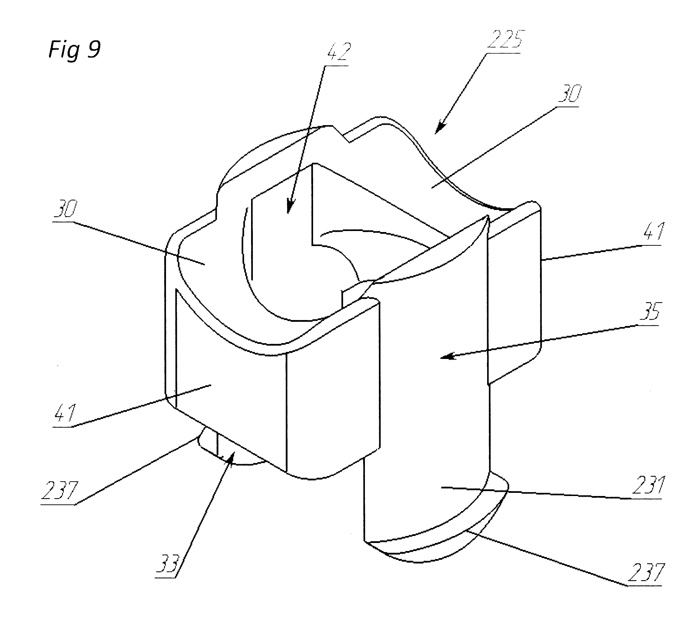

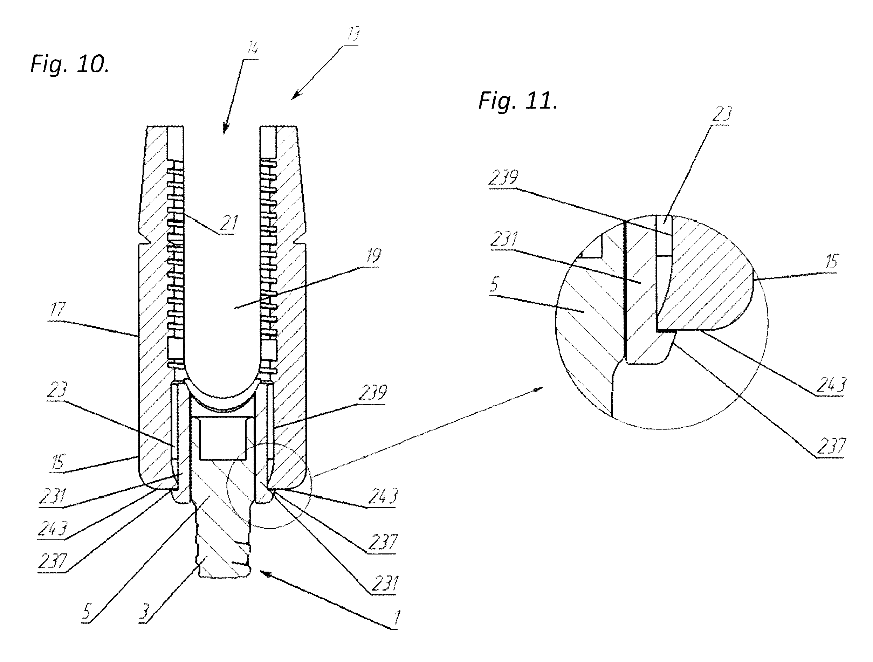

A third embodiment of the inventive pedicle anchor assembly is shown in figures 9 to 11. The third embodiment differs from the second embodiment in the design of the washer 225 and of the inner wall 239 of the axial bore 23. Elements that do not differ from those of the second embodiment are denominated by the same reference numerals as used in figures 6 to 8 and are not described again in order to avoid unnecessary repetitions.

In the third embodiment the axial extensions 231 of the washer 225 form resilient axial members, like in the second embodiment. The outer faces 35 of the axial extensions 231 are equipped with projections 237 which are located at the distal ends of the axial extensions. The inner wall 239 of the axial bore 23 does not have a projection. Instead, the rim 243 includes a projection 245 extending along the circumference of the rim and showing towards the inside of the bore 23. This projection serves as a snap-in element cooperating with the projections 237 of the axial extensions 231.

When the washer 225 is inserted into the housing 13 through the axial open end 14 so that it reaches the axial bore 23, the axial extensions 221 of the washer 225 will be compressed by the projection 245 at the rim 243 due to the shape of the projections 237 of the axial extensions 231 and the resilient properties of the material the washer is made of. When the washer is further pressed towards the bottom section 15 of the housing 13 the projections 237 of the axial extensions 231 will exit the axial bore so that the axial extensions 231 snap back and the projections 237 engage the rim 243 of the axial bore 23.

Like in the other embodiments, the inner faces 33 of the axial extensions 231 serve for holding and guiding the head 5 of the bone anchor 1 so that the bone anchor can only be pivoted in a plane that is parallel to the planes of the inner faces 33 of the axial extensions 231. Please note that in all 3 embodiments the same bone anchor 1 may be used. Therefore, the bone anchors are not described with respect to the second and third embodiments.

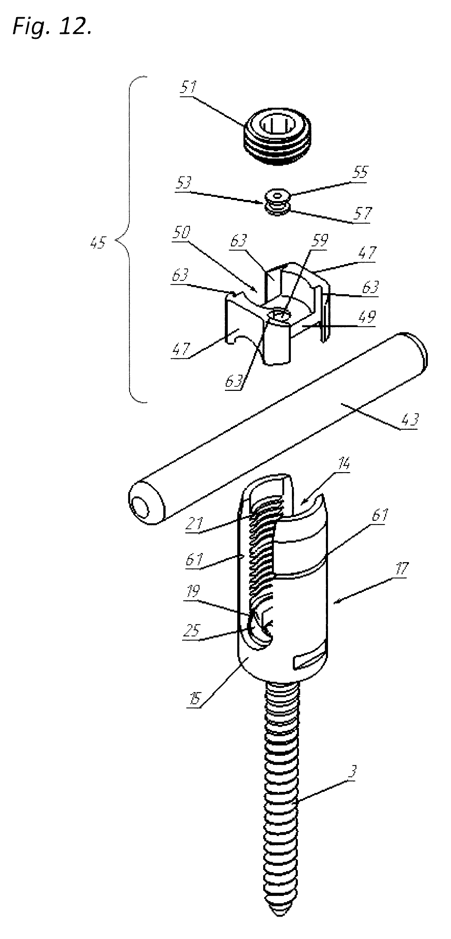

In all three embodiments, a rod 43 can be inserted into the housing 13 through the axial open end 15 and moved towards the bottom section 15 until it comes to rest on the curved surfaces 30 of the washer as it is shown in fig.12. Then, a plug element 45 may be screwed into the housing 13 for securing the rod 43 therein.

The plug element 45 comprises two wall sections 47 which are located at diametrically opposite ends of a traverse 49 connecting the two wall sections 47. The lower side of the traverse, as well as the lower side of the wall sections, shows a curvature that is adapted to the curvature of the rod 43. Moreover, an open space is formed above the traverse 49 and between the walls 47. This open space accommodates a short headless screw or threaded pin which is held inside the opening 50 by means of a retaining ring 53 with an upper section 55 that is located in a hole in the underside of the headless screw 51 and a second section 57 that is located in a hole 59 in the traverse 49. The retaining ring allows a rotational movement of the headless screw 51 about its longitudinal axis.

When the headless screw 51 is rotated about its longitudinal axis the plug element 45 moves along the axial direction of the housing 13. Thereby, the two wall sections 47 extend into the slits 19 of the housing 13 so as to prevent the plug element 45 from rotating about the longitudinal axis of the screw. At the same time, notches 63 in the wall sections 47 embrace parts of the jacket wall 17 so as to provide for guidance of the plug element 45. Moving the plug element 45 along the axial direction of the housing 13 is done, until it reaches the rod 43 so that the rod can be clamped between the washer and the plug element 45 by fastening the plug element 45 using the headless screw 51.

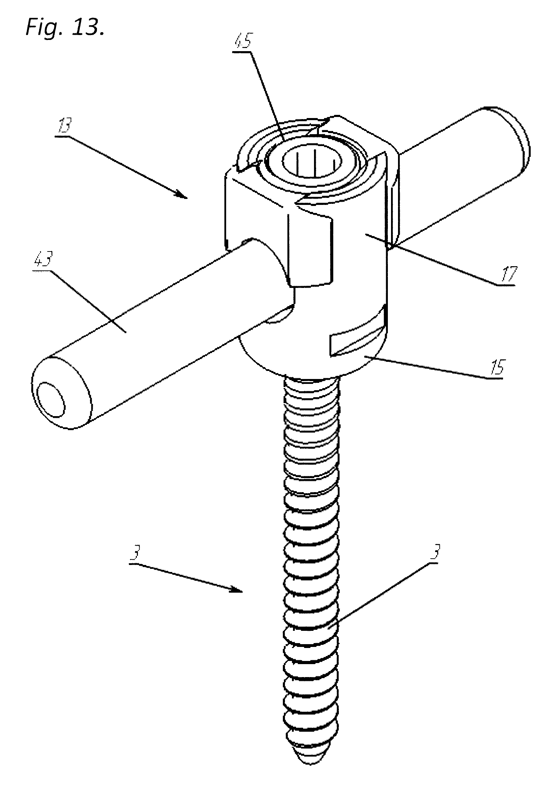

After the rod 43 has been clamped between the washer and the plug element 45 wall sections of the jacket wall of the housing 13 are broken way so as to shorten the axial length of the housing. The wall sections to be broken away are defined by a predetermined braking line 61 given by a circumferential groove which reduces the wall thickness of the jacket wall. The final state of the assembled pedicle anchor assembly, i.e. after fixing the rod and shortening the housing is shown in Figure 13.

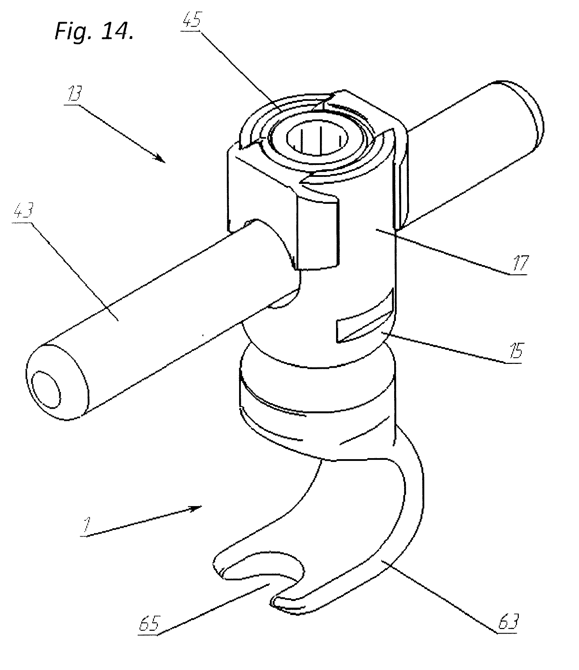

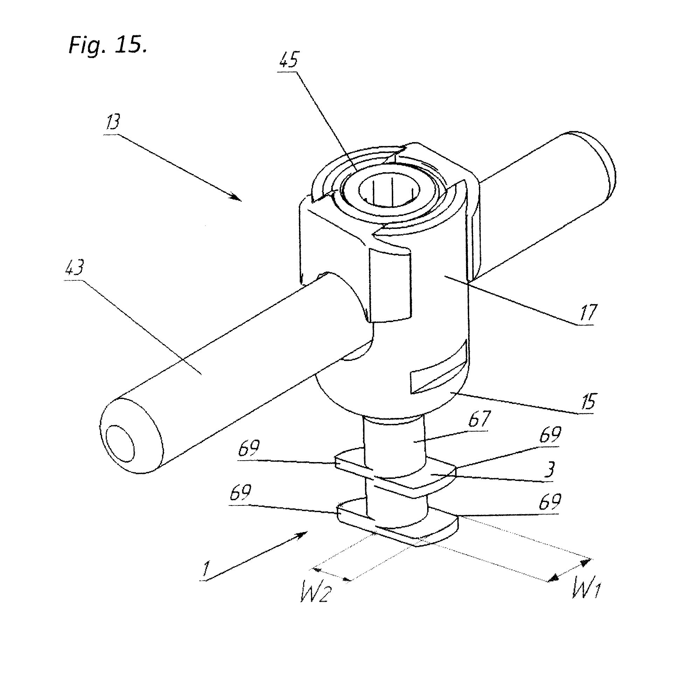

Further embodiments of the inventive pedicle anchor assembly are shown in Figures 14 and 15. Both embodiments show the pedicle anchor assembly in the assembled state after fixing the rod 43 and shortening the housing 13. The embodiments of Figures 14 and 15 differ from the embodiment shown in Figure 13 only in the bone engaging portion.

In the embodiment shown in Figure 14, instead of being implemented as a screw 3 as in the embodiment shown in Figure 13, the bone engaging portion is implemented as a hook 63 with a notch 65 in its distal end. The distal end of the hook 63 extends more or less parallel to the rod 43 secured in the housing 13.

The bone engaging portion of the embodiment shown in Fig. 15 is implemented as a cylindrical bolt 67 extending along a longitudinal axis and being equipped with plate-like protrusions 69 extending from the circumference of the bolt 67 in a direction substantially perpendicular to the longitudinal axis. In the embodiment shown in Fig. 15 these plate-like protrusions 69 each have a width that corresponds to the diameter of the bolt 67 in a first direction (width w1) and a width between 1/2 and 3/2 the diameter of the bolt 67 in a second direction (width w2) which is perpendicular to the first direction and to the longitudinal direction. All plate-like protrusions 67 are oriented along the same direction in the present embodiment.

The present invention has been described by means of special embodiments for illustrative reasons. However, modifications of these embodiments are possible. For example instead of being a screw the bone engaging portion of the bone anchor may be implemented as a hook or as a harpoon like structure. Moreover, in the shown embodiments, the head of the bone anchor has the shape of a ball with two diametrically opposite flat faces. In alternative implantations the head may comprise cylindrical faces connecting the flat faces where the cylinder axes of these cylindrical faces are parallel to the axial direction of the housing. Please note that in this implementation the cylindrical faces do not need to extend over the whole axial extensional of the head. Instead, it is enough that the cylindrical face extents over a part of the axial extensional of the head, so that the head has a barrel like shape. Then, the lower part and the upper part of the head could still be spherical. In addition, the cross section of the housing perpendicular to the axial direction does not need to be substantially circular, as is in the shown embodiments. As already mentioned, the outside cross section of the housing may be quadratic, polygonal, or even an irregular.

Since a person skilled in the art recognizes that various modifications of the shown embodiments are possible the scope of protection shall not be limited to the special embodiments described in the specification but only by the appended claims.

## Claims

1. A pedicle anchor assembly comprising:
- a bone anchor (1) being designed to anchor the assembly in a bone structure, the bone anchor (1) including a bone engaging portion (3) with a distal end and a proximal end, and a head (5) located at the proximal end of the bone engaging portion (3), where the head (5) includes two diametrically opposite sides with flat head faces (7) and has a first head width (d1) in a first direction extending between the flat head faces (7) and a second head width (d2) in a second direction perpendicular to the first direction, the second head width being larger than the first head width, and
- a housing (13) extending along an axial direction and having an open axial end (14), the housing (13) being adapted to house the head (5) of the bone anchor (1) and to receive a rod (43) through the open axial end (14), where the housing (13) includes a bottom section (15) with a seat for the head (5) of the bone anchor (1) and with an axial bore (23) which allows the engaging portion (3) to extend from the head (5) through and out of the axial bore (23), the axial bore (23) having a bore width smaller than at least the second head portion width (d2), and where the housing (13) includes a jacket wall section (17) which extends in axial direction from ore close from the bottom section (15) to the open axial end (14) and which includes slits (19) extending axially in the jacket wall section (17) from the bottom section (15) to the open axial end (14), where the slits (19) are located on diametrically opposite sides of the jacket wall section (17) so as to allow the rod (43) to be inserted into the housing (13) through the open axial end (14) and to extend through and out of the housing (13) in a direction perpendicular to the axial direction,
- a washer (25, 125, 225) with dimensions allowing it to be inserted into the housing (13) through the axial open end (14), the washer (25, 125, 225) including two axial extensions (31, 131, 231) which extend from a first side of the washer (25, 125, 225) in axial direction of the housing (13) when the washer (25, 125, 225) has been placed in the housing, the two axial extensions (31, 131, 231) each having an outer face (35) showing away from the respective other axial extension (31, 131, 231) and a flat inner face (33) showing towards the respective other axial extension (31, 131, 231), where the distance between the flat inner faces (33) of the axial extensions (31, 131, 231) is adapted to the first head width (d1) so that the flat head faces (7) can be held and guided by the flat inner faces (33) of the axial extensions (31, 131, 231) and where the shapes of and the distance between the outer faces (35) of the axial extensions (31, 131, 231) correspond to the shape and width of the axial bore (23) in the bottom section (15) of the housing (13) such that two axial extensions (31, 131, 231) can extend into or through the axial bore (23); and
- cooperating first and second snap-in connection elements ( 37, 22, 137, 141, 237, 243) where the first snap-in connection elements (37, 137, 237) are located at the washer (25, 125, 225) and at least one second snap-in connection element (22, 141, 243) is located at the housing (13).

2. The pedicle anchor assembly as claimed in claim 1, wherein the axial extensions (131, 231) of the washer are resilient axial members and the first snap-in connection elements (137, 237) are located at the outer faces (35) of the resilient axial members (131, 231) of the washer (125, 225) and at least one second snap-in connection element (141, 243) is located at the axial bore (23).

3. The pedicle anchor assembly as claimed in claim 2, wherein the first snap-in connection elements (137) are located in an axial middle section of the outer faces (35) of the resilient axial members (131) of the washer (125) and at least one second snap-in connection element (141) is located at an inside wall (139) of the axial bore (23).

4. The pedicle anchor assembly as claimed in claim 2, wherein the first snap-in connection elements (237) are located at an axial end of the outer faces (35) of the resilient axial members (231) of the washer (225) and at least one second snap-in connection element (243) is located at or formed by the rim of the axial bore (23).

5. The pedicle anchor assembly as claimed in any of the claims 2 to 4, wherein the first snap-in connection elements (137) are projections extending from the outer faces (35) of the resilient axial members (131) and the least one second snap-in connection element is a recess in the wall of the axial bore (23).

6. The pedicle anchor assembly as claimed in any of the claims 2 to 4, wherein the first snap-in connection elements (137) are recesses in the outer faces (35) of the resilient axial members (131) and the least one second snap-in connection element is a projection (141) extending from the inner wall (139) of the axial bore (23).

7. The pedicle anchor assembly as claimed in any of the claims 1 to 6, wherein the washer (25, 125, 225) has a second side with a shape that is adapted to the shape of the rod (43) to be received by the housing (13).

8. The pedicle anchor assembly as claimed in any of the claims 1 to 7, wherein cylindrical or spherical faces (9) extend between the flat faces (7) of the head (5) of the bone anchor (1), where the cylindrical or spherical faces (9) are equipped with an external thread, and wherein the housing (13) is equipped with an internal thread (21) at the inside of the jacket wall section (17), where the internal thread (21) begins at the open axial end of the housing and extends to a location at a distance from the a bottom section (15) of the housing (13) and where a thread free section (22) is formed between the internal thread (21) and the bottom section (15) of the housing (13).

9. The pedicle anchor assembly as claimed in claim 8, wherein axial length of the thread free section (22) within the jacket wall section (17) of the housing (13) is larger than an axial extension of the head (5) of the bone anchor (1).

10. The pedicle anchor assembly as claimed in any of the claims 1 to 9, wherein the jacket wall section (17) of the housing (13) is equipped with a predetermined breaking line (61) along its circumference which breaking line (61) is located in an axially mid-section of the jacket wall section (17).

11. The pedicle anchor assembly as claimed in any of the claims 1 to 10, wherein the head (5) of the bone anchor (1) is equipped with a socket (11) for a driving key.

12. The pedicle anchor assembly as claimed in claim 11, wherein the washer (25, 125, 225) has an opening (42) that allows a driving key to reach the socket (11) in the head (5) of the bone anchor (1).

13. The pedicle anchor assembly as claimed in any of the claims 1 to 12, further comprising a plug element (45) which includes a pin (51) with an external thread corresponding to the internal thread (21) of the jacket wall section (17) of the housing (13).

14. The pedicle anchor assembly as claimed in claim 13, wherein the plug element (45) includes two opposing wall sections (47) and a traverse (49) connection the wall sections (47), where the wall sections (47) are dimensioned such that they fit into the slits of the jacket wall section (17) of the housing (13) and wherein the pin (51) is rotatably secured to the traverse.

15. The pedicle anchor assembly as claimed in claim 13 or claim 14, wherein the traverse (49) has an underside with a shape that is adapted to the shape of the rod (43) to be received by the housing (17).
